# EUROPEAN PATENT APPLICATION

(11) **EP 3 782 728 A1**
(43) Date of publication of application: **24.02.2021**
(21) Application number: 20175943.8
(22) Date of filing: 21.05.2020
(51) Int. Cl.: B01J 27/138, B01J 31/02, B01J 31/04, C08G 63/183

(54) **CATALYST FOR USE IN ESTERIFICATION REACTION AND METHOD FOR CATALYZING ESTERIFICATION REACTION**

(30) Priority: 21.06.2019 US 201962864506 P; 07.10.2019 TW 108136282
(71) Applicant: Borica Co., Ltd., Taipei City 100 (TW)
(72) Inventor: HSIEH, Chia-Wei, Taipei City 100 (TW); CHUANG, Yun-Hsuan, Taipei City 100 (TW); SHIH, Chun-Han, Taipei City 100 (TW); TSUO, Fu-Ming, Taipei City 100 (TW)
(74) Representative: Mewburn Ellis LLP

(57) **Abstract**

A catalyst for use in esterification reaction is provided. The catalyst is formed by reacting a mixture including at least one first compound and at least one second compound. The at least one first compound is a metal alkoxide, an inorganic metal salt, a metal carboxylate salt, an inorganic metal compound, or a combination thereof, and each foregoing compound has titanium, aluminum, zirconium, hafnium, zinc, or bismuth. The at least one second compound is an alpha hydroxyl acid, an alkyl ester formed by an alpha hydroxyl acid and an alcohol, an alkyl amide formed by an alpha hydroxyl acid and an amine, an amino acid, an alkyl ester formed by an amino acid and an alcohol, an alkyl amide formed by an amino acid and an amine, or a combination thereof.

## Description

### BACKGROUND

### Field of Invention

The present disclosure relates to a catalyst for use in an esterification reaction and a catalytic method.

### Description of Related Art

Esters are widely used in today's life. Common catalysts used for an esterification reaction include tin-based catalysts (for example, dibutyltin dilaurate (DBTDL), tin octoate, or tin acetylacetonate), titanium-based catalyst (for example, titanium alkoxide, titanium lactate, or titanium citrate) or antimony-based catalyst (for example, antimony trioxide or antimony glycolate). The reaction rate of tin-based catalysts is fast, and the appearance of the ester product is usually satisfying. However, most of organotin compounds are considered as highly toxic substances, and thus European Union (EU) has legislated to restrict their application. The same situation also occurs with antimony-based catalysts. For example, in Japan, the use of antimony-based catalysts in hot-fill bottles of polyethylene terephthalate (PET) is restricted. For current commercially available titanium-based catalysts, although being non-toxic with good reactivity, these catalysts easily get discolored and hydrolyzed during production of polyester chips and the forming process of articles later, thus resulting defective products.

During continuous production of polyesters, such as polybutylene terephthalate (PBT), catalyst hydrolysis will lead to periodic shutdown of production line to clean up the precipitate, which not only consumes a lot of manhour and reduces production capacity. In addition, environmental awareness has risen in recent years, and the demand for environmentally friendly catalysts cannot be underestimated. In view of the foregoing, there is an urgent need to develop better catalysts to improve the above problems.

### SUMMARY

One aspect of the present disclosure provides a catalyst for use in an esterification reaction. The catalyst is formed by reacting a mixture including at least one first compound and at least one second compound. The at least one first compound is a metal alkoxide, an inorganic metal salt, a metal carboxylate salt, an inorganic metal compound, or a combination thereof, which have titanium, aluminum, zirconium, hafnium, zinc, or bismuth. The at least one second compound is an alpha hydroxyl acid, an alkyl ester formed by an alpha hydroxyl acid and an alcohol, an alkyl amide formed by an alpha hydroxyl acid and an amine, an amino acid, an alkyl ester formed by an amino acid and an alcohol, an alkyl amide formed by an amino acid and an amine, or a combination thereof.

In some embodiments, the metal alkoxide having titanium, aluminum, zirconium, hafnium, zinc, or bismuth, has a formula of M(OR₁)(OR₂)(OR₃)ₓ(OR₄)_{y}, R₁, R₂, R₃, and R₄ are independently C₂-C₈ alkyl groups, when M is titanium, zirconium, or hafnium, x = 1 and y = 1, when M is aluminum or bismuth, x = 1 and y = 0, and when M is zinc, x = 0 and y = 0.

In some embodiments, the inorganic metal salt having titanium, aluminum, zirconium, hafnium, bismuth, or zinc includes chloride, nitrate, sulfate, or a combination thereof, the metal carboxylate salt having titanium, aluminum, zirconium, hafnium, bismuth, or zinc includes acetate, propionate, or a combination thereof, and the inorganic metal compound having titanium, aluminum, zirconium, hafnium, bismuth, or zinc includes a metal oxide, a metal hydroxide, or a combination thereof.

In some embodiments, the alpha hydroxyl acid includes glycolic acid, lactic acid, citric acid, malic acid, tartaric acid, or a combination thereof.

In some embodiments, the alkyl ester formed by the alpha hydroxyl acid and the alcohol has C₂-C₁₈ alkyl groups, the alkyl amide formed by the alpha hydroxyl acid and the amine has C₂-C₁₈ alkyl groups, the alkyl ester formed by the amino acid and the alcohol has C₂-C₁₈ alkyl groups, and the alkyl amide formed by the amino acid and the amine has C₂-C₁₈ alkyl groups.

In some embodiments, based on a weight of the catalyst, a total weight percentage concentration of titanium, aluminum, zirconium, hafnium, bismuth and zinc in the catalyst is 0.01 wt% - 50 wt%.

In some embodiments, a molar ratio of the at least one first compound to the at least one second compound is 1:0.1 - 1:6.

Other aspect of the present disclosure provides a method of catalyzing an esterification reaction. The method includes the following operations: providing a mixture, wherein the mixture including at least one alcohol and at least one acid; and adding the catalyst mentioned in any one of previous embodiments into the mixture to form an ester.

In some embodiments, the at least one alcohol includes ethylene glycol, propylene glycol, butylene glycol, pentanediol, hexanediol, glycerol, isosorbide, cyclohexane dimethanol, pentaerythritol, neopentyl glycol, trimethanol propane, lactic acid, citric acid, or a combination thereof.

In some embodiments, the least one acid includes terephthalic acid, isophthalic acid, trimellitic acid, trimellitic anhydride,
naphthalene-2,6-dicarboxylic acid, adipic acid, succinic acid, malonic acid, suberic acid, sebacic acid , maleic acid, fumaric acid, lactic acid, citric acid, citraconic acid, mesaconic acid, itaconic acid, or a combination thereof.

### DETAILED DESCRIPTION

One aspect of the present disclosure provides a catalyst for use in an esterification reaction. The catalyst is formed by reacting a mixture including at least one first compound and at least one second compound.

Specifically, the at least one first compound is a metal alkoxide, an inorganic metal salt, a metal carboxylate salt, an inorganic metal compound, or a combination thereof, and each foregoing compound has titanium, aluminum, zirconium, hafnium, zinc, or bismuth. In some embodiments, the metal alkoxide having titanium, aluminum, zirconium, hafnium, zinc, or bismuth, has a formula of M(OR₁)(OR₂)(OR₃)ₓ(OR₄)_{y}, wherein R₁, R₂, R₃, and R₄ are independently C₂-C₈ alkyl groups. When M is titanium, zirconium, or hafnium, x = 1 and y = 1. When M is aluminum or bismuth, x = 1 and y = 0. When M is zinc, x = 0 and y = 0. In certain embodiments, R₁, R₂, R₃, and R₄ are independently linear alkyl or branched alkyl. For example, R₁, R₂, R₃, and R₄ include but are not limited to methyl, ethyl, n-propyl, isopropyl, n-butyl, sec-butyl, isobutyl, tert-butyl, n-pentyl , tert-amyl, neopentyl, isopentyl, sec-pentyl, 3-pentyl, hexyl, heptyl, octyl, nonyl or decyl.

In some embodiments, the inorganic metal salt having titanium, aluminum, zirconium, hafnium, bismuth, or zinc includes chloride, nitrate, sulfate, or a combination thereof, the metal carboxylate salt having titanium, aluminum, zirconium, hafnium, bismuth, or zinc includes acetate, propionate, or a combination thereof, and the inorganic metal compound having titanium, aluminum, zirconium, hafnium, bismuth, or zinc includes a metal oxide, a metal hydroxide, or a combination thereof.

Specifically, the at least one second compound is an alpha hydroxyl acid, an alkyl ester formed by an alpha hydroxyl acid and an alcohol, an alkyl amide formed by an alpha hydroxyl acid and an amine, an amino acid, an alkyl ester formed by an amino acid and an alcohol, an alkyl amide formed by an amino acid and an amine, or a combination thereof. In various embodiments, the alpha hydroxy acid includes glycolic acid, lactic acid, citric acid, malic acid, tartaric acid, or a combination thereof. The amino acid includes aspartic acid, glycine, arginine, histidine, lysine, glutamate, alanine, serine, threonine, aspartame, glutamate, cysteine, proline, valine, isoleucine, leucine, methionine, amphetamine, tyrosine, tryptophan, or a combination thereof.

In some embodiments, the alkyl ester formed by the alpha hydroxyl acid and the alcohol has C₂-C₁₈ alkyl groups. The alkyl amide formed by the alpha hydroxyl acid and the amine has C₂-C₁₈ alkyl groups. The alkyl ester formed by the amino acid and the alcohol has C₂-C₁₈ alkyl groups. The alkyl amide formed by the amino acid and the amine has C₂-C₁₈ alkyl groups. The aforementioned C₂-C₁₈ alkyl groups may be linear alkyl groups or branched alkyl groups, but it is not limited thereto.

In some embodiments, based on a weight of the catalyst, a total weight percentage concentration of titanium, aluminum, zirconium, hafnium, bismuth and zinc in the catalyst is 0.01 wt% - 50 wt%, for example, 0.01 wt%, 1 wt%, 5 wt%, 10 wt%, 20 wt%, 30 wt%, 40 wt%, or 50 wt%. In some embodiments, a molar ratio of the at least one first compound to the at least one second compound is 1:0.1 - 1:6, for example, 1: 0.1, 1:1, 1:2, 1:3, 1:4, 1:5, or 1:6. The total weight percentage concentration and the molar ratio between the ranges disclosed herein can improve the catalyst reaction performance and accelerate the esterification reaction rate without catalyst hydrolysis.

Other aspect of the present disclosure provides a method of catalyzing an esterification reaction. The method includes providing a mixture, wherein the mixture including at least one alcohol and at least one acid; and adding the catalyst mentioned in any one of previous embodiments described above into the mixture to form esters.

In some embodiments, the at least one alcohol includes a diol, a polyol, or a combination thereof. For example, the diol includes ethylene glycol, propylene glycol, butylene glycol, pentanediol, hexanediol, glycerin, isosorbide, cyclohexanedimethanol, neopentyl glycol, or a combination thereof. The polyol includes pentaerythritol, trimethanol propane, lactic acid, citric acid, or a combination thereof.

In other embodiments, the at least one acid includes a monobasic acid, a dibasic acid, a polybasic acid, or a combination thereof. For example, the monobasic acid includes trimellitic anhydride. The dibasic acid includes terephthalic acid, isophthalic acid, naphthalene-2,6-dicarboxylic acid, adipic acid, succinic acid, malonic acid, suberic acid, sebacic acid, maleic acid, fumaric acid diacid, citraconic acid, mesaconic acid, itaconic acid, or a combination thereof. The polyacid includes trimellitic acid, lactic acid, citric acid, or a combination thereof.

The following embodiments are used to describe specific aspects of the present disclosure and enable one skilled in the art in the technical field to implement the present disclosure. However, the following examples should not be used to limit the present disclosure.

### Experiment 1: Synthesis of catalyst

The experimental procedures of Example 1 were briefly described as follows. First, to a round-bottomed flask equipped with a stirrer and a condenser, 3.50 g (12 mmol) of titanium tetra-isopropoxide (TIPT, provided by BORICA CO., LTD. under the trademark of "Tytan") and 6.00 g (24 mmol) of Mixture 1 (the mixture of citric acid, monobutyl citrate, dibutyl ester, and tributyl ester, the acid value of Mixture 1 = 200 mgKOH/g) to form a reaction mixture, wherein the ratio of the total mole of Mixture 1 and the mole of titanium tetra-isopropoxide is 2:1. Next, after stirring the above reaction mixture for 30 minutes, the catalyst of Example 1 was obtained.

The catalysts of Examples 2 to 9 can also be prepared by the above steps. Compared with Example 1, Examples 2 to 9 used the same or different first compound, second compound, and molar ratio to prepare the catalysts. The detailed experimental conditions were summarized in Table 1 below. The molar ratio and metal content (wt%) of the reaction mixture were listed in Table 1, wherein the molar ratio was the mole number of the compound with Ti in the first compound : the mole number of the compound without Ti in the first compound : the mole number of the second compound; the metal content was the weight percentage of different metals in the first compound. The first compound may be zirconium tetra-n-propoxide (TNPZ), zinc chloride, aluminum tri-s-butoxide, zirconium n-butoxide (TNBZ), or titanium tetra-ethoxide (ET). The second compound may be Mixture 1, Mixture 2 (the mixture of citric acid, monoethyl citrate, diethyl ester and triethyl ester, the acid value of Mixture 2 = 200 mgKOH / g), or ethyl lactate. Zirconium tetra-n-propoxide, titanium tetra-ethoxide, and zirconium n-butoxide were provided by BORICA CO., LTD. under the trademark of "Tytan."

**Table 1**

| Example | First compound | Second compound | Molar ratio | Metal content (wt %) |
|---|---|---|---|---|
| 1 | TIPT | Mixture 1 | 1:0:2 | Ti:6.9 |
| 2 | TIPT/TNPZ | Mixture 2 | 1:1:2 | Ti: 3.25 |
| | | | | Zr: 7.91 |
| 3 | TIPT/ZnCl₂ | Mixture 2 | 1:1:2 | Ti: 3.60 |
| | | | | Zn: 8.58 |
| 4 | TIPT/ Aluminum tri-s-butoxide | Mixture 2 | 1:1:2 | Ti: 3.45 |
| | | | | Al: 5.40 |
| 5 | TNBZ | Mixture 1 | 0:1:2 | Zr: 9.83 |
| 6 | ZnCl₂ | Ethyl lactate | 0:1:2 | Zn: 16.53 |
| 7 | Aluminum tri-s-butoxide | Ethyl lactate | 0:1:2 | Gelled |
| 8 | TIPT | Ethyl lactate | 1:0:2 | Ti: 9.65 |
| 9 | ET | Mixture 1 | 1:0:2 | Ti: 7.49 |

### Experiment 2: Catalyst performance test

The catalysts of the above Examples 1-9, dibutyltin dilaurate (DBTDL), titanium tetrabutoxide (TNBT), bis(2-hydroxyethyl) terephthalate (BHET, reagent grade, purity 85%) were used to simulate PET polymerization reaction, and a rapid measurement of the reactivity of Examples 1-9 was performed. The experimental procedures were as follows. First, BHET was melted at a temperature of 107 to 110 °C, and then 10 ppm (calculated as metal atoms) of the catalyst (Sn dosage of DBTDL is 285 ppm) was added to form a reaction mixture. The reaction mixture was continuously stirred until the mixture become uniform. Next, the reaction mixture was cooled, and the cooled reaction mixture was ground to a powder. 10 mg of the ground powder was placed in a 40uL standard aluminum crucible equipped with a lid, and the thermogravimetric loss of the sample was measured by using a thermogravimetric analyzer / differential thermal scanning analyzer (available from Mettler Toledo Corporation, TGA / DSC 3+ STAR). The experimental parameters were as follows: (i) The first stage: the sample was heated from 50 °C to 280 °C, the heating rate is 10 K / min, and the nitrogen flow rate is 50 mL / min; (ii) The second stage: the sample was heated at 280 °C for 50 minutes, and the nitrogen flow rate is 50 mL / min.

The inflection point temperature (°C) experimental results obtained after the experiment of the condensation reaction of BHET with the addition of catalysts (Examples 1 to 9) were listed in Table 2.

**Table 2**

| Example | First compound | Second compound | Inflection point (°C) |
|---|---|---|---|
| 1 | TIPT | Mixture 1 | 268.24 |
| 2 | TIPT/TNPZ | Mixture 2 | 253.81 |
| 3 | TIPT/ ZnCl₂ | Mixture 2 | 268.55 |
| 4 | TIPT/Aluminum tri-s-butoxide | Mixture 2 | 268.33 |
| 5 | Zirconium n-butoxide | Mixture 1 | 276.06 |
| 6 | ZnCl₂ | Ethyl lactate | 275.92 |
| 7 | Aluminum tri-s-butoxide | Ethyl lactate | Gelled |
| 8 | TIPT | Ethyl lactate | 262.78 |
| 9 | ET | Mixture 1 | 268.42 |

| Comparative Example | Catalyst | | Inflection point (°C) |
|---|---|---|---|
| 1 | DBTDL (285 ppm Sn) | | 258.57 |
| 2 | TNBT (10 ppm Ti) | | 256.14 |

The lower the inflection point temperature was, the faster the rate of the condensation reaction of BHET was, and the better the effect of the catalyst was. From Table 2, it can be seen that, compared with Comparative Examples 1 to 2, the inflection point temperatures of Examples 1 to 6 and 8 to 9 were in a good range. This result confirmed that these catalysts had excellent catalytic effect.

### Experiment 3: Catalyst hydrolysis test

The catalysts prepared in Experimental 1 (Examples 1 to 5 and 9) were used for hydrolysis test to discuss the stability of these catalysts in water. The experimental method was briefly described as follows. First, after mixing the catalyst of Example 1 and water at a weight ratio of 9: 1, a sample can be obtained. Next, this sample was heated at 200 °C for 1 hour, and a hydrolysis resistance test was performed. The appearance of the samples before and after heating was summarized in Table 3 below.

The samples of Examples 2 to 5 and 9 can also be prepared by the above steps. The sample of Comparative Example 3 in Table 3 was titanium n-butoxide (TNBT) and water at a weight ratio of 9:1.

**Table 3**

| | Sample | Before heating | After heating |
|---|---|---|---|
| Comparative Example 3 | TNBT + water | turbid | turbid |
| Example 1 | Catalyst 1 + water | clear | clear |
| Example 2 | Catalyst 2 + water | clear | clear |
| Example 3 | Catalyst 3 + water | clear | clear |
| Example 4 | Catalyst 4 + water | clear | clear |
| Example 5 | Catalyst 5 + water | clear | clear |
| Example 9 | Catalyst 9 + water | clear | clear |

Before heating, the samples of Examples 1 to 5 and 9 showed clear when visually observed, indicating that the catalyst was not hydrolyzed. After heating at 200 °C for 1 hour, the sample remained clear when visually observed, thus confirming that the catalysts prepared in Experimental 1 of the present disclosure were not hydrolyzed in water, so it can maintain good catalytic performance when catalyzing an esterification reaction. In contrast, when the catalyst was mixed with water, the sample of Comparative Example 3 appeared cloudy before being heated, indicating that the catalyst of Comparative Example 3 had no hydrolysis resistance at all, resulting in poor catalytic performance.

In summary, the present disclosure provides a catalyst for an esterification reaction and a method for catalyzing an esterification reaction. The catalyst will not undergo hydrolysis reaction after heating, so it is beneficial for the esterification reaction and maintains good catalytic performance. At the same time, it solves the problem that the continuous production must be shut down to clean the equipment regularly due to catalyst hydrolysis, and the production efficiency can be enhanced. It is worth noting that the catalyst used in the present disclosure not only has excellent catalytic speed in the esterification reaction, but also is a non-toxic catalyst. Therefore, it is an environmentally friendly catalyst that conforms to the concept of sustainable operation.

## Claims

1. A catalyst for use in an esterification reaction, the catalyst formed by reacting a mixture comprising at least one first compound and at least one second compound, the catalyst is **characterized in that**
the at least one first compound is a metal alkoxide, an inorganic metal salt, a metal carboxylate salt, an inorganic metal compound, or a combination thereof, which have titanium, aluminum, zirconium, hafnium, zinc, or bismuth; and
the at least one second compound is an alpha hydroxyl acid, an alkyl ester formed by an alpha hydroxyl acid and an alcohol, an alkyl amide formed by an alpha hydroxyl acid and an amine, an amino acid, an alkyl ester formed by an amino acid and an alcohol, an alkyl amide formed by an amino acid and an amine, or a combination thereof.

2. The catalyst of claim 1, the catalyst is **characterized in that** the metal alkoxide having titanium, aluminum, zirconium, hafnium, zinc, or bismuth, has a formula of M(OR₁)(OR₂)(OR₃)ₓ(OR₄)_{y}, R₁, R₂, R₃, and R₄ are independently C₂-C₈ alkyl groups, when M is titanium, zirconium, or hafnium, x = 1 and y = 1, when M is aluminum or bismuth, x = 1 and y = 0, and when M is zinc, x = 0 and y = 0.

3. The catalyst of claim 1 or claim 2, the catalyst is **characterized in that** the inorganic metal salt having titanium, aluminum, zirconium, hafnium, bismuth, or zinc comprises chloride, nitrate, sulfate, or a combination thereof, the metal carboxylate salt having titanium, aluminum, zirconium, hafnium, bismuth, or zinc comprises acetate, propionate, or a combination thereof, and the inorganic metal compound having titanium, aluminum, zirconium, hafnium, bismuth, or zinc comprises a metal oxide, a metal hydroxide, or a combination thereof.

4. The catalyst of any one of claims 1-3, the catalyst is **characterized in that** the alpha hydroxyl acid comprises glycolic acid, lactic acid, citric acid, malic acid, tartaric acid, or a combination thereof.

5. The catalyst of any one of claims 1-4, the catalyst is **characterized in that** the alkyl ester formed by the alpha hydroxyl acid and the alcohol has C₂-C₁₈ alkyl groups, the alkyl amide formed by the alpha hydroxyl acid and the amine has C₂-C₁₈ alkyl groups, the alkyl ester formed by the amino acid and the alcohol has C₂-C₁₈ alkyl groups, and the alkyl amide formed by the amino acid and the amine has C₂-C₁₈ alkyl groups.

6. The catalyst of any one of claims 1-5, the catalyst is **characterized in that**, based on a weight of the catalyst, a total weight percentage concentration of titanium, aluminum, zirconium, hafnium, bismuth and zinc in the catalyst is 0.01 wt% - 50 wt%.

7. The catalyst of any one of claims 1-6, the catalyst is **characterized in that** a molar ratio of the at least one first compound to the at least one second compound is 1:0.1 - 1:6.

8. A method of catalyzing an esterification reaction, the method **characterized by** comprising:
providing a mixture, wherein the mixture comprises at least one alcohol and at least one acid; and
adding the catalyst of any one of claims 1-7 into the mixture to form an ester.

9. The method of claim 8, the method is **characterized in that** the at least one alcohol comprises ethylene glycol, propylene glycol, butylene glycol, pentanediol, hexanediol, glycerol, isosorbide, cyclohexane dimethanol, pentaerythritol, neopentyl glycol, trimethanol propane, lactic acid, citric acid, or a combination thereof.

10. The method of any one of claims 8-9, the method is **characterized in that** the least one acid comprises terephthalic acid, isophthalic acid, trimellitic acid, trimellitic anhydride, naphthalene-2,6-dicarboxylic acid, adipic acid, succinic acid, malonic acid, suberic acid, sebacic acid, maleic acid, fumaric acid, lactic acid, citric acid, citraconic acid, mesaconic acid, itaconic acid, or a combination thereof.
